# EUROPEAN PATENT SPECIFICATION

(11) **EP 1 446 173 B1**
(45) Date of publication and mention of the grant of the patent: **30.09.2015**
(21) Application number: 02804027.7
(22) Date of filing: 21.11.2002
(51) Int. Cl.: A61M 25/00

(54) **A MULTILUMEN CATHETER AND METHODS FOR MAKING THE CATHETER**
MULTILUMEN-KATHETER UND VERFAHREN ZUR HERSTELLUNG DES KATHETERS
CATHETER MULTI-LUMIERE ET PROCEDES DE FABRICATION

(30) Priority: 21.11.2001 US 331882 P
(43) Date of publication of application: 18.08.2004
(60) Divisional application: 12188505.7
(73) Proprietor: Medical Components, Inc., Harleysville, PA 19438 (US)
(72) Inventor: MADISON, Anthony J., Lansdale, PA 19446 (US); SCHWEIKERT, Timothy M., Levittown, PA 19054 (US); SCHON, Donald A., Paradise Valley, AZ 85021-7243 (US)
(74) Representative: Patentanwaltskanzlei Matschnig & Forsthuber OG
(86) International application number: PCT/US2002/037444
(87) International publication number: WO 2003/045464

(56) References cited:
- EP-A- 0 332 366
- EP-A- 0 711 574
- EP-A1- 0 453 234
- US-A- 4 309 994
- US-A- 4 405 313
- US-A- 5 100 395
- US-A- 5 380 276
- US-A- 5 549 579
- US-A- 5 599 304
- US-A- 5 683 640
- US-A- 5 800 414
- US-A- 5 807 311
- US-A- 6 001 079
- US-B1- 6 190 349

## Description

### Related Applications

This application claims benefit of U.S. Provisional Application Serial No. 60/331,882, filed November 21, 2001, entitled "Multilumen Catheter."

### Field of the Invention

The present invention relates generally to multilumen catheter assemblies, and more particularly to multilumen catheter assemblies having a smooth, rounded unitary catheter portion for positioning at a vessel wall insertion site and independent, free floating catheter tubes for positioning within an area to be catheterized. Such a catheter is disclosed in the US 6190349.

### Background of the Invention

Catheters for the introduction or removal of fluids may be located in various venous locations and cavities throughout the body for the introduction or removal of fluids. Such catheterization may be performed by using a single catheter having multiple lumens. A typical example of a multiple lumen catheter is a dual lumen catheter in which one lumen introduces fluids and one lumen removes fluids. Catheterization may also be performed by using separate, single lumen catheters inserted through two different incisions into an area to be catheterized. Such multiple catheter assemblies are known as Tesio catheters. Procedures are also known as described in U.S. Patent No. 5,624,413 for inserting two wholly independent single lumen catheters into a vessel through a single insertion site.

Generally, to insert any catheter in a blood vessel, the vessel is identified by aspiration with a long hollow needle in accordance with the Seldinger technique. When blood enters a syringe attached to the needle, indicating that the vessel has been found, a thin guide wire is then introduced, typically through a syringe needle or other introducer device, into the interior of the vessel. The introducer device is then removed leaving the guide wire within the vessel. The guide wire projects beyond the surface of the skin.

At this point, several options are available to a physician for catheter placement. The simplest is to pass a catheter into the vessel directly over the guide wire. The guide wire is then removed leaving the catheter in position within the vessel. However, this technique is only possible in cases where the catheter is of a relatively small diameter, made of a stiff material and not significantly larger than the guide wire, for example, for insertion of small diameter dual lumen catheters. If the catheter to be inserted is significantly larger than the guide wire, a dilator device is first passed over the guide wire to enlarge the hole. The catheter is then passed over the guide wire, and the guide wire and dilator are removed.

In the case of an individual, single-lumen catheter typically used in multiple catheter assemblies (e.g., a Tesio catheter), a physician may use an introducer sheath. If a Tesio catheter is used for hemodialysis, for example, each catheter is inserted in two separate veins, such as the femoral vein. Alternatively, each catheter may be inserted in two different locations of the same vein, such as the internal jugular vein as noted above. The introducer sheath is simply a large, stiff thin-walled tube, which serves as a temporary conduit for the permanent catheter which is being placed. Tearaway sheaths are also available which split apart for easier removal. The introducer sheath is positioned by placing a dilator device inside of the introducer and passing both the dilator and the introducer together into the vessel over a guide wire. The guide wire, left in the vessel after insertion as described above, and the dilator are then removed, leaving the thin-walled introducer sheath in place. The catheter is placed through the introducer sheath. Each of the catheters in the assembly is typically subcutaneously secured within the patient's body by a cuff located in a subcutaneous tunnel, or by otherwise externally affixing the catheter to the body.

The Tesio catheter may also be inserted, in accordance with the technique described in U.S. Patent No. 5,624,413, as noted above, through a single insertion point using a sheath into the vessel. The Tesio catheter, once inserted in the vessel, is then tunneled separately through the patient in two subcutaneous tunnels for securement of the external, proximal portions of the catheter.

The Tesio double catheter assembly, while comfortable for the patient, due to its soft durometer, and very effective for hemodialysis, typically requires multiple procedures and incisions for insertion and/or for tunneling, which increase the attendant risks of the catheterization procedure. Further, in the case of side-by-side placement of two catheter tubes through a single insertion site in a vessel, while minimizing the number of procedures, can present a potential for leakage between the catheter tubes at the point where the catheter tubes pass into the vessel.

However, Tesio catheter assemblies provide catheters which are capable of independent movement within the vessel. Such catheters present several advantages over unitary multilumen catheters formed of a single internally divided tube when in the vessel. Because the individual tubes of a Tesio double catheter assembly are independently movable at their fluid outlets, it is possible to provide fluid intake and/or return flow around the entire circumference of the distal ends of the catheter tubes. In addition, if one tube becomes blocked, or otherwise requires replacement, it can be removed independently of the other tube. Further, the softer durometer of such catheters, which are typically made of a silicone or a similar material, reduces the risk of vessel wall damage. The 360° circumferential flow provides a more stable tube within the vessel, which is less likely to be suctioned against the vessel wall due to a pressure differential, as occasionally occurs in the use of some side-by-side multi-lumen catheters.

U.S. Patent No. 5,718,692, issued to Schon, et al., ("the Schon catheter") describes a self-retaining double catheter system in which each catheter can be subcutaneously secured without the use of fabric tissue ingrowth cuffs or external suturing as a result of the placement of a retaining sleeve surrounding both individual catheters in a multiple catheter assembly to hold the catheters together at the location of the sleeve. The individual catheters are permanently linked in one portion by a hub for self-anchoring under the skin, as an alternative to requiring a fabric stabilizing cuff, such that such cuffs are optional. The distal ends are longitudinally prespaced by an appropriate distance to avoid recirculation. While this device requires only one incision, it requires two subcutaneous tunnels in order to facilitate the self-retaining feature. This catheter provides independently movable distal ends within the vessel and 360° circumferential flow in the manner of a standard Tesio. Further, since the retaining sleeve is located outside the vessel when in place to provide the self-retaining feature, at the point of entry into the vessel, the catheters are side-by-side in the manner of a standard Tesio catheter, and there still remains the potential risk of blood leakage between the catheters at the vessel site.

U.S. Patent No. 5,947,953 discloses a splittable multiple catheter assembly that has a hub and at least two fully independent catheter tubes which are initially releasably joined together, for example, by a breakable membrane. A single subcutaneous tunnel may be used in inserting the catheter, and the catheter tubes are at least partially separated by splitting the catheter tubes prior to insertion into a vessel. As a result, the portions of the catheter within the vessel are capable of independently moving and having 360° circumferential flow from the distal portion of each tube. The catheter may be secured using standard securement means such as suturing, ingrowth or other available securement devices.

A further multiple catheter assembly is described in U.S. Patent No. 5,776,111 for use in acute Tesio catheterizations. The assembly includes two independent single lumen catheters joined at a location by a generally flat disc that may be attached to the surface of a patient's skin to secure the assembly in an acute procedure. The distal ends are prespaced to avoid recirculation.

There is a need in the art for a multiple catheter assembly and a need for making such a catheter assembly which can provide the advantages of the above-mentioned multi-lumen catheters with respect to easy insertion through a single tunneling procedure and which can prevent the potential risk of leakage at the site of vessel entry, but which can still provide the advantage of multiple catheter assemblies with respect to independent movement within a vessel and good flow properties.

### Summary of the Invention

The present invention is a multilumen catheter assembly which provides easy catheter insertion by a single tunneling procedure and assists the prevention of leakage at the site of vessel entry through use of a unitary, outer wall configuration as further disclosed in claim 1. The catheter assembly also provides independent, free floating movement within the vessel through use of separate, distal end catheter tubes and provides efficient fluid flow properties within lumens of the catheter assembly.

In one aspect of the present invention, the multilumen catheter assembly includes a unitary portion having an outer wall, a distal end, a proximal end, and a plurality of lumens extending longitudinally through the unitary portion. In this aspect, the catheter assembly also includes a plurality of distal end tubes, each defining a longitudinally extending lumen therethrough, where the lumens of the distal end tubes are each in fluid communication with a respective lumen of the unitary portion, and the distal end tubes are capable of independent movement with respect to each other. In this aspect of the invention, the outer wall of the unitary portion, the outer wall of the distal end tubes, and the lumens, can have various shapes in cross section, such as but not limited to a circular, semi-circular, or oval shape. The unitary portion, the distal end tubes, and the lumens, can also have a different shape or configuration at different points along a respective longitudinal length of each.

In another aspect of the present invention, the multilumen catheter assembly includes a unitary catheter having a rounded exterior surface, a first lumen and a second lumen extending longitudinally therethrough, a distal end and a proximal end; and a first distal end tube defining a first longitudinally extending passageway and a second distal end tube defining a second longitudinally extending passageway, where the first and the second distal end tubes extend distally from the distal end of the unitary catheter, the first passageway in the first distal end tube being in fluid communication with the first lumen, the second passageway in the second distal end tube being in fluid communication with the second lumen and the first and second distal end tubes are capable of independent movement with respect to each other.

In another aspect of the present invention, the distal end tubes are releasably attached to one another over a portion, or the entirety, of their longitudinal length, allowing the first and the second distal end tubes to be split, through use of minimal force, and separate over any portion of their longitudinal length.

In another aspect of the present invention, the first and the second distal end tubes are releasably attached to one another, and possibly having an outer wall with semi-circular cross section, from the distal end of the unitary catheter to a bonding point located between the distal end of the unitary catheter and the distal end of the catheter assembly, allowing the first and the second distal end tubes to be split, through use of minimal force, to any longitudinal point between the distal end of the unitary catheter and the transition point, the first and the second distal end tubes being separate from one another, and possibly circular in cross section, from the transition point to the distal end of the catheter assembly, providing the first and the second distal end tubes with individual and independent movement with respect to each other from the transition point to the distal end of the catheter assembly. In this aspect of the present invention, the unitary catheter can have a generally oval cross section.

In another aspect of the invention, the multilumen catheter assembly includes two catheter tubes, each having a round lumen extending longitudinally therethrough and each having an exterior of generally semi-circular cross sectional shape. In this aspect, the catheter assembly also includes a hub securing the two catheter tubes in juxtaposed alignment with respect to one another, wherein the tubes in juxtaposed alignment together have a generally oval shape in cross section.

The present invention also provides a method for making a multilumen catheter assembly as further disclosed in claim 22, the method includes forming a unitary catheter tube having a proximal portion and a distal portion, the distal portion terminating at a distal end and the proximal portion terminating at a proximal end, the unitary catheter tube having a first lumen and a second lumen, the first lumen and the second lumen each extending longitudinally through the unitary catheter tube. The formed unitary catheter tube is then split longitudinally along the distal portion to form a first distal end tube and a second distal end tube. The unitary catheter tube, the distal end tubes, and lumens within each, can each then be finished, if desired, to have one or more configurations, or cross sectional shapes, over a respective longitudinal length of each.

In one aspect of the present invention, the distal end tubes are then releasably attached along any portion, or an entirety, of their longitudinal lengths. In another aspect, the distal end tubes are releasably attached from a transition point (the point of transition, after splitting, between the unitary catheter and the first and the second distal end tubes) to a bonding point located between the transition point and the distal end of the catheter assembly.

### Brief Description of the Drawings

The foregoing summary, as well as the following detailed description of the invention, will be better understood when read in conjunction with the appended drawings. For the purpose of illustrating the invention, there are shown in the drawings certain embodiments of the present invention. It should be understood, however, that the invention is not limited to the precise arrangements and instrumentalities shown. In the drawings, the same reference numerals are employed for designating the same elements throughout the several figures. In the drawings:
Figure 1 is a top view of a multilumen catheter assembly in accordance with a first embodiment of the present invention;
Figures 1a and 1b are enlarged cross-sectional views of the multilumen catheter assembly of Figure 1 taken along lines 1a-1a and 1b-1b, respectively;
Figures 2a through 2e are enlarged cross-sectional views of alternative embodiments of the multilumen catheter assembly of Figure 1 taken along line 1b-1b;
Figure 2f is an enlarged cross-sectional view of an alternative embodiment of the multilumen catheter assembly of Figure 1 taken along line 1a-1a;
Figure 3 is an enlarged cross-sectional view of the hub of the multilumen catheter assembly of Figure 1;
Figure 4 is a top view of a multilumen catheter assembly in accordance with a second embodiment of the present invention;
Figures 4a, 4b and 4c are enlarged cross-sectional views of the multilumen catheter assembly of Figure 4 taken along lines 4a-4a, 4b-4b, and 4c-4c, respectively;
Figure 5 is a top view of a multilumen catheter assembly in accordance with a third embodiment of the present invention;
Figures 5a and 5b are enlarged cross-sectional views of the multilumen catheter assembly of Figure 5 taken along lines 5a-5a and 5b-5b, respectively;
Figure 6 is a top view of a multilumen catheter assembly; Figures 6a and 6b are enlarged cross-sectional views of the multilumen catheter assembly of Figure 6 taken along lines 6a-6a and 6b-6b, respectively;
Figure 7 is a top view of a multilumen catheter assembly;
Figures 7a and 7b are enlarged cross-sectional views of the multilumen catheter assembly of Figure 7 taken along lines 7a-7a and 7b-7b, respectively;
Figure 8 is a top view of a multilumen catheter assembly; Figures 8a and 8b are enlarged cross-sectional views of the multilumen catheter assembly of Figure 8 taken along lines 8a-8a and 8b-8b, respectively;
Figure 9 is a top view of a multilumen catheter assembly; Figures 9a and 9b are enlarged cross-sectional views of the multilumen catheter assembly of Figure 9 taken along lines 9a-9a and 9b-9b, respectively;
Figure 10a is a top view of a unitary catheter tube for use in making a multilumen catheter assembly according to one embodiment of the invention;

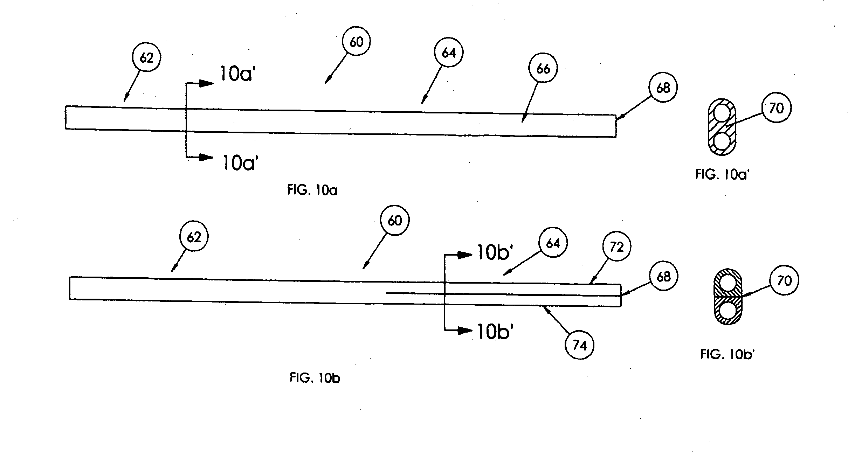

Figure 10a' is an enlarged cross-sectional view of the unitary catheter tube of Figure 10a taken along line 10a'-10a';
Figure 10b is a top view of the unitary catheter tube of Figure 10a which has been split at a distal end to form distal end tubes; and
Figure 10b' is an enlarged cross-sectional view of the unitary catheter tube of Figure 10b taken along line 10b'-10b'.

### Detailed Description of the Invention

In describing the embodiments of the invention illustrated in the drawings, specific terminology will be used for the sake of clarity. However, the invention is not intended to be limited to the specific terms so selected, it being understood that each specific term includes all technical equivalents operating in similar manner to accomplish similar purpose. It is understood that the drawings are not drawn exactly to scale. In the drawings, similar reference numbers are used for designating similar elements throughout the several figures.

The following describes particular embodiments of the invention. However, it should be understood, based on this disclosure, that the invention is not limited to the embodiments detailed herein. Generally, the following disclosure refers to double lumen catheter assemblies, although catheter assemblies having three or more lumens and/or distal end tubes are within the scope of the invention. Further, the methods described below for making the catheter assemblies of the present invention are also applicable to making catheter assemblies having more than two lumens and/or distal end tubes. It is only for reasons of convenience that the following description refers to two lumen embodiments of the present invention.

The multilumen catheter assemblies of the present invention are inserted into an area of a body of a patient to be catheterized for removing and introducing fluids to the body. The catheter assemblies of the present invention are secured to a fixed location in or on the patient body, such as a subcutaneous area, before the catheter assembly is properly inserted and positioned in the area to be catheterized. This method is particularly preferred for chronic catheterization. Alternatively, in an acute catheterization, the catheter assemblies of the present invention may be secured to an external surface of the body before or after the catheter assembly is properly inserted and positioned in the area to be catheterized.

The multilumen catheter assemblies of the present invention can be adapted for use in various applications in which bodily fluids, medicaments, or other solutions are introduced into and removed from the body, such as perfusion, infusion, plasmapheresis, hemodialysis, chemotherapy, and the like. The catheter assemblies of the present invention are particularly suitable for chronic hemodialysis and apheresis. The area to be catheterized is preferably a blood vessel, such as an internal jugular vein, but may be any suitable area within the body. Other areas in which the catheter assemblies may be used include other blood vessels, including the femoral and subclavian veins, any abscess cavity, post-operative cavity, the peritoneal cavity, and other areas of the body including intra-abdominal, sub-diaphragmatic and subhepatic areas. It is understood that the above-referenced areas are exemplary, and that the catheter assemblies of the present invention may be used to remove or introduce fluids to various areas to be catheterized.

The embodiments of the present invention shown in the figures are particularly useful for intake, or removal, of blood to be purified from a blood vessel, such as the internal jugular vein, and introduction of purified blood into the same vessel. The blood can be purified by any suitable hemodialysis apparatus (not shown), attached in communication with lumens of the disclosed catheter assemblies. The catheter assemblies of the present invention may also be used to introduce medication or other fluids, including glucose or saline solutions, into the body.

For purposes of describing the embodiments of the present invention shown in the figures, the catheter assemblies will be described with respect to an application of hemodialysis; more specifically, an application for purifying blood flowing through an internal jugular vein. However, it is understood that the catheter assemblies of the present invention can be configured and adapted, by increasing or decreasing a size (diameter or length) and/or number of distal end tubes and/or lumens in the respective catheter assembly, so that the catheter assembly can be beneficially used for other medical applications in which fluids are introduced into and/or removed from the body.

### A First Embodiment

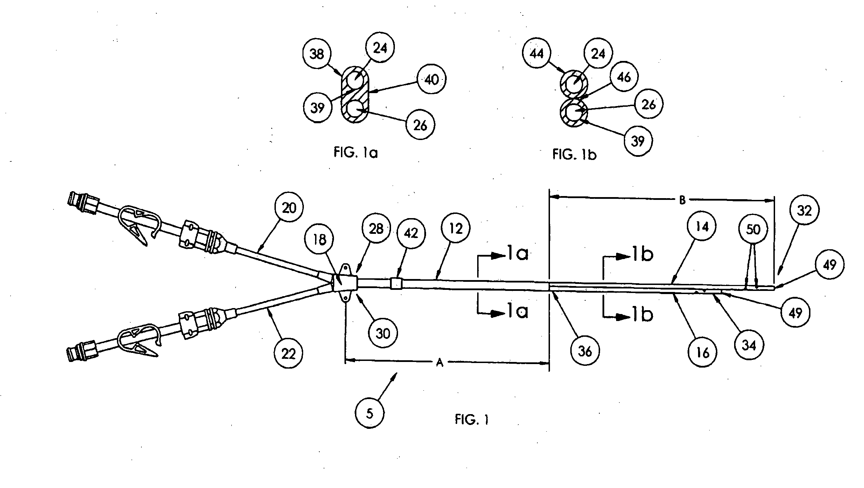

Figure 1 illustrates one embodiment of the present invention, where a catheter assembly 5 has at least two lumens. The illustration of two lumens is exemplary, and the scope of the invention encompasses catheters having more than two lumens.

The catheter assembly 5 includes a unitary catheter 12, a first distal end tube 14, a second distal end tube 16, a hub 18, and a first and a second extension tube 20, 22. The multilumen catheter assembly 5 includes a first lumen 24 and a second lumen 26 extending longitudinally therethrough (see Figures 1a and 1b), the first lumen 24 and the second lumen 26 having proximal ends 28, 30, respectively, terminating within the hub 18, and distal ends 32, 34, respectively, terminating at distal ends 32, 34 of the first and the second distal end tubes 14, 16.

The first lumen 24 is continuous with and through the first extension tube 20, and the second lumen 26 is continuous with and through the second extension tube 22, both by connection at the hub 18. The first and the second extension tubes 20, 22 lead to a proximal end of the catheter assembly 5, through which the materials entering and exiting the patient enter and/or exit the catheter assembly 5. The words "proximal" and "distal" refer to directions away from and closer to, respectively, the inserted end of the catheter assembly 5.

An imaginary transition-point 36 exists at a point along a longitudinal length of the first and the second lumens 24, 26. The catheter assembly 5 can be provided (manufactured) so that the first distal end tube 14 and the second distal end tube 16 are splittable (releasably attached) or separate at their respective distal ends 32, 34, and splittable or separate from their distal ends 32, 34 up to the transition-point 36 (over a longitudinal length in Figure 1 generally denoted by Arrow "B"). Splittable is defined as releasably attached, meaning the first and the second distal end tubes 14, 16 are adhered, bonded, fused, or otherwise attached, so that only minor force is necessary to pull apart, or split, the tubes 14, 16. Minor force could be defined as approximately one to five pounds of force.

The portion of the catheter assembly 5 between the proximal ends 28, 30 of the first and the second lumens 24, 26 and the transition-point 36 includes the unitary catheter 12 (over a longitudinal length in Figure 1 generally denoted by Arrow "A"). An exterior of the unitary catheter 12 includes a smooth, curved, and generally convex surface without ridges or grooves. Any of various shapes providing a smooth, curved, and generally convex surface without ridges are contemplated in the present invention.

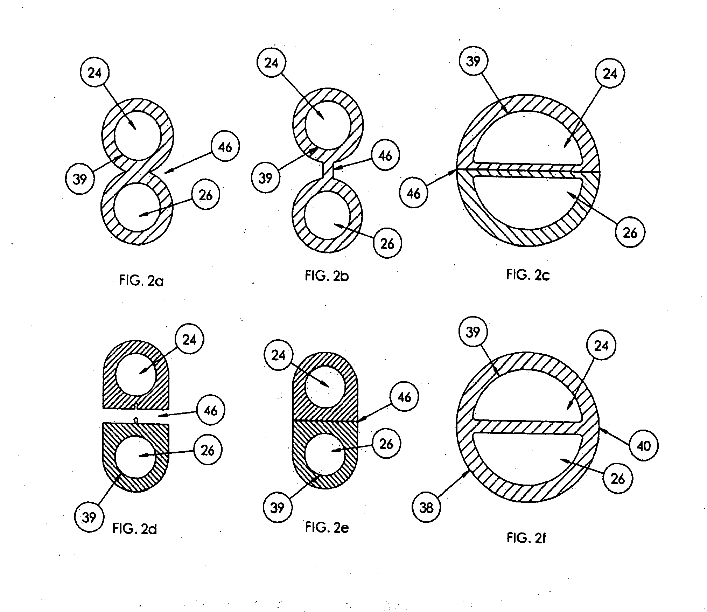

As shown in Figure 1a, the cross-section of the unitary catheter 12 can be generally oval in shape (outer configuration), Figure 1a illustrating in cross-section a generally oval shaped outer wall 38, with the first and the second lumens 24, 26 having a circular cross-section (as shown by outer walls 39 of the first and the second lumens 24, 26) and a first and second lumen separating member 40. Another non-limiting example of a unitary catheter 12 cross-section is shown in Figure 2f. The lumens 24, 26 within either an oval unitary catheter 12 outer wall 38 configuration (such as Figure 1a), or a more rounded, or even a circular unitary catheter 12 outer wall 38 configuration (such as Figure 2f), could be any one of various shapes, such as but not limited to circular, semi-circular, oval, triangular, square, elliptical, or kidney-bean shaped.

A cuff 42 can be located at a point along the unitary catheter 12. Cuffs 42 are known in the art and provide a surface onto which internal tissue may adhere to stabilize the catheter assembly 5 within the patient.

The transition-point 36 can be located exactly at a half-way point of the lumens 24, 26 (i.e., at a point half-way between the proximal ends 28, 30 and the distal ends 32, 34 of the lumens 24, 26, the half-way point measured using the longest of the lumens 24, 26 if the lumens 24, 26 are of different lengths, such as in Figure 1). The transition-point 36, however, could be located at any point along the longitudinal length of the catheter assembly 5. In Figure 1, for instance, the transition-point 36 is located at the mid-point between the distal end 32 and the proximal end 28 of the first lumen 24.

In another aspect of the invention, the transition-point 36 is located at a point along the longitudinal length of the lumens 24, 26 such that a longitudinal length of the separate or splittable portion of the lumens 24, 26 (i.e., the first and the second distal end tubes 14, 16, as generally denoted by Arrow "B") is greater than a longitudinal length of the unitary catheter 12 (as generally noted by Arrow "A"). In this alternative embodiment, the longitudinal length of the separate or splittable portion of the lumens 24, 26 (the Arrow "B" portion) is measured using the longer of the lumens 24, 26. Alternatively stated, the first and the second lumens 24, 26 are separate or splittable from one another (i.e., releasably attached) from their respective distal ends 32, 34 to a point on the lumens 24, 26 that is at least one-half of the length of the lumens 24, 26 measured from the distal end of the longest lumen to the respective proximal end of the longest lumen.

In the above mentioned embodiments, it is noted that the proximal ends 28, 30 of the lumens 24, 26 may occur at different locations in various catheters. It is within the scope of the present invention to incorporate, in the dimensional aspects of length disclosed above, all locations where the proximal ends 28, 30 could be said to occur in catheters known in the art, disclosed herein, or to be developed.

The smooth, curved, generally convex exterior surface of the unitary catheter 12 passes through and remains positioned at a vessel wall insertion site during insertion of the catheter assembly 5 into a patient. A vessel wall seals quite well around the smooth, curved exterior surface of the unitary catheter 12, as shown in cross-section in Figure 1a, and seals particularly well with a ligature. Since the exterior of the unitary catheter 12 provides a good seal at the insertion site, the risk of blood loss around the catheter assembly 5 at the insertion site is minimized. This is especially true relative to a situation where individual lumens pass through and remain located at a vessel wall insertion site, as a tight seal around individual lumens having, generally, a figure-8 configuration is difficult (the reference to a figure-8 configuration referring to the exterior cross-section of two, circular and individual lumens, such as that shown in Figure 1b).

The first and the second distal end tubes 14, 16 extend distally from the unitary catheter 12 at the transition point 36, with the first and the second lumens 24, 26 having continuous fluid communication therethrough. The first and the second distal end tubes 14, 16 preferably have outer surfaces continuous with the outer wall 38 of the unitary catheter 12, and are capable of independent movement when split from one another.

The first and the second distal end tubes 14, 16 can be well known in the art, or newly developed. In Figure 1b, the first and the second distal end tubes 14, 16 are defined by circular outer walls 44 of the first and the second distal end tubes 14, 16, the first and the second lumens 24, 26, the circular outer walls 39 of the first and the second lumens 24, 26, and a junction point 46. Figures 2a through 2e illustrate cross-sections of alternative embodiments of splittable first and second distal end tubes 14, 16 (i.e., alternatives to the Figure 1b embodiment). The junction point 46 can be created by weak adhesives (Figures 2a, 2c, & 2e), by molding (Figure 2b), by tongue-in-groove arrangement (Figure 2d), or by other methods enabling the distal end tubes 14, 16 to be split through use of minimal force, such that the juncture of the lumens is not fixed.

The first and the second distal end tubes 14, 16, and the first and the second lumens 24, 26 within the distal end tubes 14, 16, have a generally circular cross section in the Figure 1 embodiment of the present invention, as shown in Figure 1b. The first and the second lumens 24, 26 are circular, as shown by the outer walls 39, since circular cross sections are most conducive to fluid flow properties. However, other shapes such as D-shaped passageways and/or lumens (Figure 2c), oval, triangular, square, elliptical, kidney-bean shaped passageways and/or lumens, or other configurations are also within the scope of the invention (some of which are shown in Figures 2a through 2e). Further, while the distal end tubes 14, 16 and the lumens 24, 26 are preferably identical in cross section, it is within the scope of the invention to vary the size, shape and/or configuration of the distal end tubes 14, 16 and/or the lumens 24, 26 such that smaller distal end tubes and/or lumens, or varying types of lumens and distal end tubes may be used for other applications, such as an addition of a third, smaller lumen and corresponding distal end tube for introduction of medication.

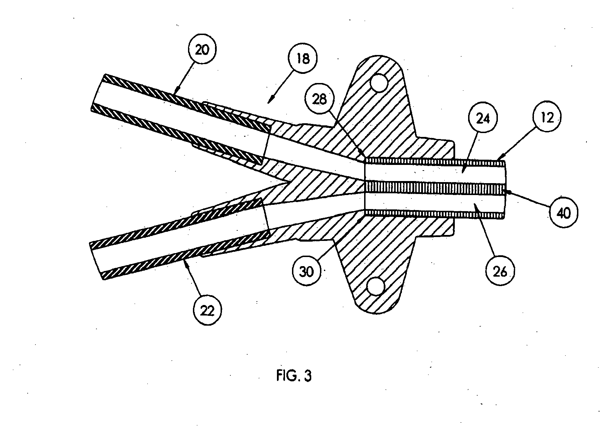

Figure 3 illustrates a cross-section of the hub 18 of Figure 1, the hub 18 providing a reinforced area for termination of the proximal ends 28, 30 of the first and the second lumens 24, 26, and for termination of distal ends of the extension tubes 20, 22. The hub could also include one or more suture wings for securing the catheter assembly to the body, if desired. Use of other hub configurations, as well as use of a detachable hub, is also envisioned for the present invention.

### A Second Embodiment

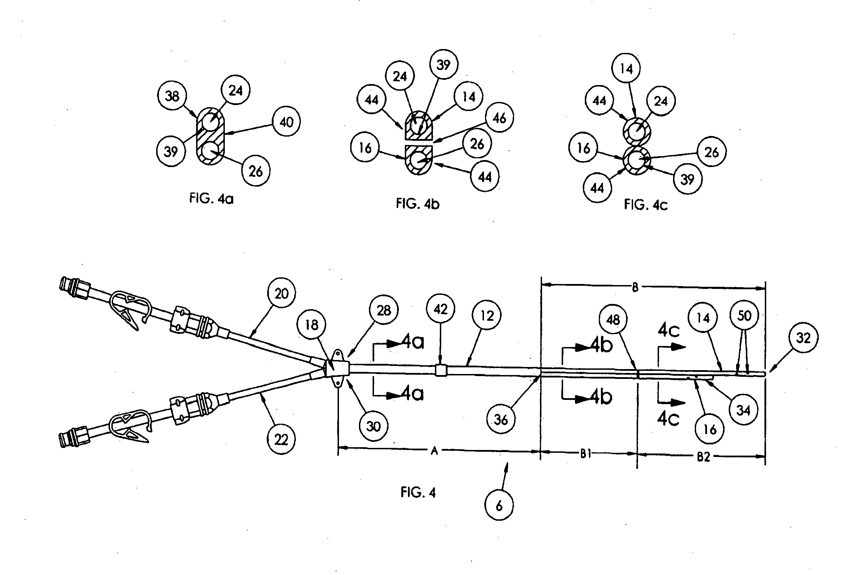

Figure 4 illustrates a catheter assembly 6, which is a second embodiment of the present invention, an embodiment having additional limitations relative to the catheter assembly 5 of Figure 1. The catheter assembly 6 of Figure 4 is primarily distinguished from the catheter assembly 5 of Figure 1 over the portion of the catheter assembly 6 between the distal ends 32, 34 and the transition-point 36 (over the portion of the longitudinal length of the catheter assembly 6 generally denoted by Arrow "B" in Figure 4). In one aspect of the Figure 4 embodiment, the transition-point 36 is located at a point along the longitudinal length of the lumens 24, 26 such that the length of the longer of the first and the second distal end tubes 14, 16 (the Arrow "B" portion) is greater than the length of the unitary catheter 12 (the Arrow "A" portion).

In the Figure 4 embodiment, the outer walls 44 of the first and the second distal end tubes 14, 16 are releasably attached from the transition point 36 to an imaginary bonding point 48 (over a longitudinal length generally denoted by Arrow "B1" in Figure 4). The releasable attachment of the first and the second distal end tubes 14, 16, from the transition point 36 to the bonding point 48 allows the first and the second distal end tubes 14, 16 to be split, through use of minimal force, to any selected longitudinal point between the transition point 36 and the bonding point 48.

Between the transition point 36 and the bonding point 48, the cross section of the first and the second distal end tubes 14, 16 (Figure 4b) illustrates that the outer configuration of the first and the second distal end tubes 14, 16 over the Arrow "B1" length resembles that of the unitary catheter 12 (i.e., smooth, curved, and generally oval shaped). Figure 4b also illustrates that the first and the distal end tubes 14, 16, when individually viewed, are semi-circular in shape, each have a generally "D" shaped outer wall 44, with a junction point 46 of weak adhesive between the flat side portions of the "D" shaped outer walls 44, the weak adhesive providing splittability through use of minimal force. In Figure 4b, the flat side portions face each other, and are identical to each other so that a cannulating portion of the catheter assembly 6 retains a rounded, generally oval cross section.

The flat side portions, when placed back to back to provide an overall oval outer configuration for the distal end tubes, assist in preventing blood tracking between the tubes, as no rounded or grooved surfaces exist between the tubes for blood to travel. The flat side portions of the tubes allow the tubes to fit back together (if separate, independent tubes are not desired) as if the tubes were never separated, and are still unitary in configuration.

The catheter assembly 6 includes adhesive, or a splittable membrane, to provide releasable attachment between the tubes 14, 16. The adhesive extends longitudinally between and joins the opposite, generally flat side portions of the first and second distal end tubes 14, 16 (Figure 4b). The adhesive (not shown in the figures) can extend longitudinally along a central line of the flat side portions of the tubes 14, 16 to provide dimensional stability. However, the adhesive could extend between edges of the flat side portions, or between other regions of the flat side portions, or the rounded wall portions of the tubes 14, 16.

The adhesive, or splittable membrane, performs multiple functions. First, the membrane joins the tubes 14, 16 so that the tubes 14, 16 can be easily manipulated, particularly where the membrane is unbroken. Where the membrane is completely intact, the catheter assembly 6 can be manipulated as a single catheter (e.g., the unitary catheter 12). Second, the membrane allows the first and the second distal end tubes 14, 16 to be at least partially longitudinally split apart from each other, without damaging the outer walls 44 of the tubes 14, 16, to allow independent movement of the split portions in the vessel or other area to be catheterized. The membrane is constructed to split easily when the first and the second tubes 14, 16 are separated from each other, thereby tearing or splitting before the opposing forces exerted on the tubes 14, 16 reach a level sufficient to cause damage thereto. However, the membrane should be sufficiently strong to resist tearing during normal handling of the catheter assembly 6.

From the bonding-point 48, to the distal ends 32, 34 of the first and the second lumens 24, 26, the first and the second distal end tubes 14, 16 are separate (unattached) and independent (over a longitudinal length generally denoted by Arrow "B2" in Figure 4). In one aspect of the present invention, the cross section of the first and the second distal end tubes 14, 16 (Figure 4c) illustrates that the outer walls 44 of the first and the second distal end tubes 14, 16 over the Arrow "B2" length are rounded. As shown in Figures 4a, 4b, and 4c, the outer walls 39 of the first and the second lumens 24, 26 illustrate a circular cross section for the lumens 24, 26.

### A Third Embodiment

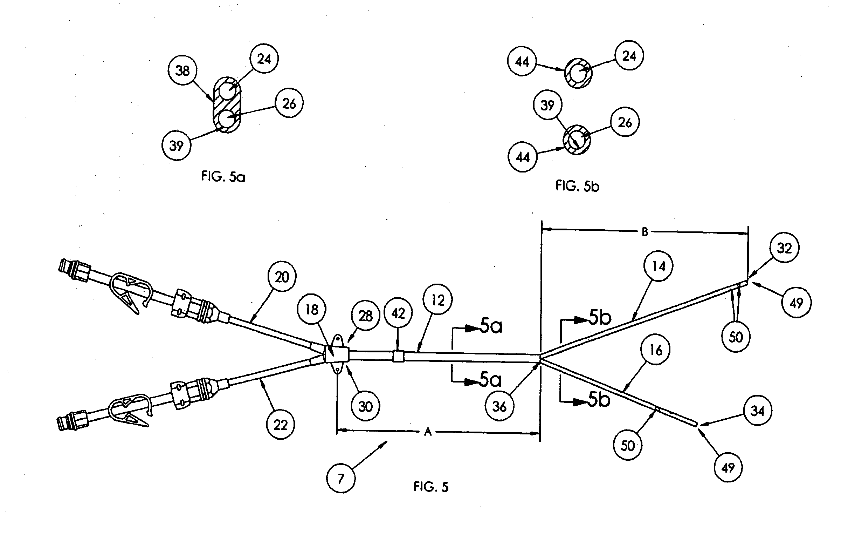

Figure 5 illustrates a catheter assembly 7, which is a third embodiment of the present invention. The catheter assembly 7 of Figure 5 is primarily distinguished from the catheter assembly 5 of Figure 1 over the portion of the catheter assembly 7 between the distal ends 32, 34 and the transition-point 36 (over the portion of the longitudinal length of the catheter assembly 7 generally denoted by Arrow "B" in Figure 5).

In the Figure 5 embodiment, the outer walls 44 of the first and the second distal end tubes 14, 16 are separate (unattached) and independent from the transition point 36 to the distal ends 32, 34 (over the entire Arrow "B" longitudinal length in Figure 5), as shown in Figure 5b. The cross section of the first and the second distal end tubes 14, 16 (Figure 5b) illustrates that the outer walls 44 of the first and the second distal end tubes 14, 16 over the Arrow "B" length, as well as the outer walls 39 of the first and the second lumens 24, 26, are rounded or circular.

The portion of the catheter assembly 7 between the proximal ends 28, 30 of the first and the second lumens 24, 26 and the transition-point 36 includes the unitary catheter 12 (over a longitudinal length in Figure 5 generally denoted by Arrow "A"). As in the embodiments of Figures 1 and 4, the exterior of the unitary catheter 12 includes a smooth, curved, and generally convex surface. As shown in Figure 5a, the cross-section of the unitary catheter 12 is generally oval in shape, Figure 5a illustrating in cross-section the generally oval shaped outer wall 38 of the unitary catheter 12 and the circular shaped outer walls 39 of the first and the second lumens 24,26.

### A Fourth Embodiment

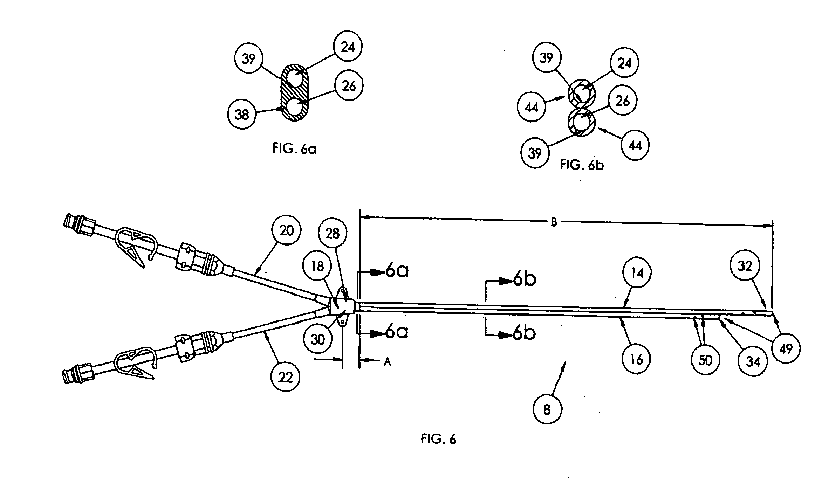

Figure 6 illustrates a catheter assembly 8, which not part of the present invention. The catheter assembly 8 of Figure 6 does not include a distinctive unitary catheter 12 portion, but rather includes the first and the second distal end tubes 14, 16 extending substantially the entire length of the catheter assembly 8 (over the longitudinal length generally denoted by Arrow "B" in Figure 6). Although the catheter assembly 8 of Figure 6 does not include a distinctive unitary catheter 12, a proximal portion of the first and the second distal end tubes 14, 16 (through and including termination of the proximal ends of the first and the second distal end tubes 14, 16 within the hub 18) includes an outer configuration having a smooth, rounded, and generally oval shaped perimeter. The hub 18 is similar to that shown in cross section in Figure 3.

As shown in Figure 6a, the cross section of the catheter assembly 8 over that portion of its longitudinal length generally denoted by Arrow "A" in Figure 6, is generally oval in shape, Figure 6a illustrating the generally oval shaped outer wall 38 and the circular shaped outer walls 39 of the first and the second lumens 24, 26. In the Figure 6 embodiment of the invention, the cross section of the Arrow "A" portion of the catheter assembly 8 could include a single, oval shaped portion (as shown in Figure 6a) or two semi-circular ("D" shaped) distal end tubes 14, 16, placed back to back (i.e., with the flat portions of the semi-circle adjoining, as shown in Figure 2e and 4b) with the flat portions permanently bonded, with perhaps a suitable adhesive, to one another.

The outer walls 44 of the first and the second distal end tubes 14, 16 can be separate (unattached) and independent over the Arrow "B" portion of the longitudinal length of the catheter assembly 8 of Figure 6, or the first and the second distal end tubes 14, 16 can be releasably attached (splittable) over this length. In either case, the cross section of the first and the second distal end tubes 14, 16, as shown in Figure 6b, illustrates that the outer walls 44 of the first and the second distal end tubes 14, 16, as well as the outer walls 39 of the first and the second lumens 24, 26, are rounded or circular.

Further, it is envisioned that the first and the second distal end tubes 14, 16 could have alternative features, such as but not limited to those shown in Figures 2a through 2e, or as described above with regard to the first embodiment of the present invention.

### A Fifth Embodiment

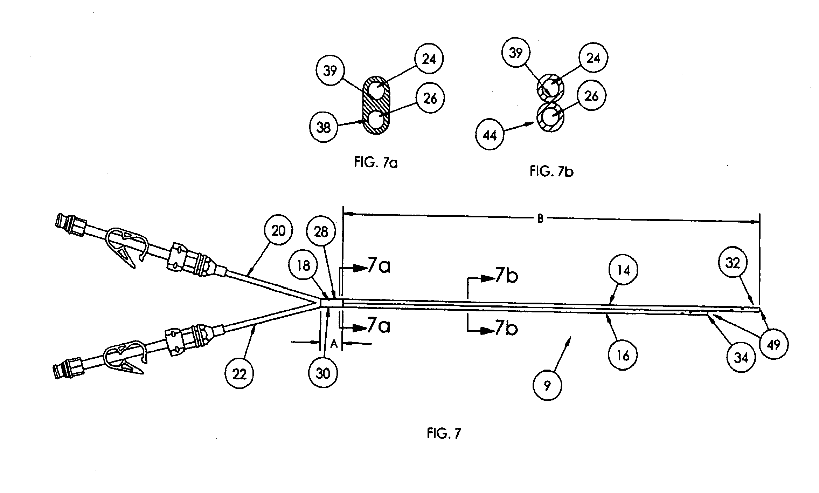

Figure 7 illustrates a catheter assembly 9, which is not part of the present invention. The catheter assembly 9 of Figure 7 is similar to the catheter assembly 8 of Figure 6, distinguished only by incorporation of an alternative hub 18.

A hub provides a reinforced area for the proximal ends 28, 30 of the first and the second lumens 24, 26, respectively, to communicate with distal ends of extension tubes. The hub also provides a means to secure one or more lumens to one another, thereby limiting capability of the lumens to be separated or to be splittable. Accordingly, the present invention contemplates use of any hub type device accomplishing the above, either known in the art or to be developed, including detachable hub devices.

As known in the art, hub 18 may be sealed, such as by bonding, adhering, heat molding, or through other attachment, to a distal end of an extension tube (or extender) and a proximal end of a tube or lumen. In one aspect of the invention, extension tubes are proximal portions of two separate lumens which are coated with an outer layer, perhaps by heat molding, to form a unitary body portion of the assembly. Accordingly, the extension tubes are continuous with the proximal ends of the lumens, and the hub 18 can be simply molded or otherwise adhered around the proximal ends of the lumens and around the distal ends of the extension tubes. In another aspect of the invention, the hub 18 (outer layer) is molded or otherwise adhered around some midpoint portion of a first and a second tube or lumen, creating extension tubes and a catheter assembly from one set of tubes or lumens. If either aspect, above, is the embodiment of the hub 18 incorporated into the catheter assembly 9 of Figure 7, it is understood that the proximal end 28, 30 of the first and the second lumens 24, 26 occurs at the point where the extension tubes 20, 22 diagonally diverge from the hub 18.

### A Sixth Embodiment

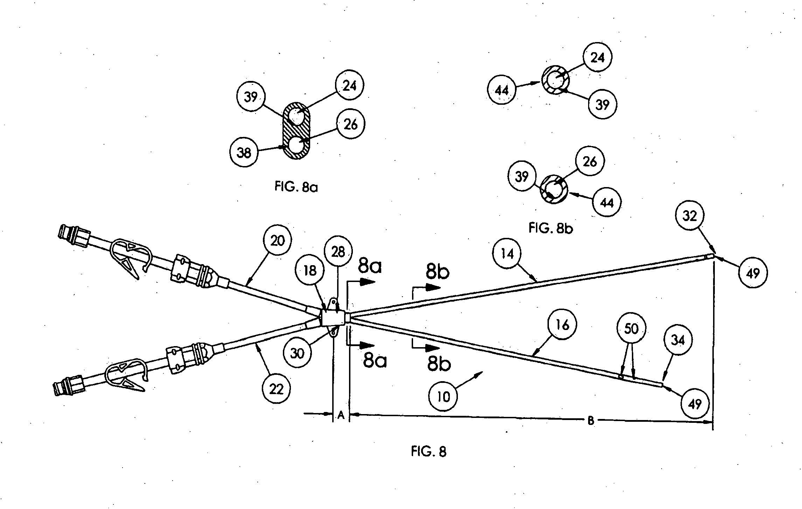

Figure 8 illustrates a catheter assembly 10, which is not part of the present invention. The catheter assembly 10 of Figure 8 is similar to the catheter assembly 8 of Figure 6, but includes first and second distal end tubes 14, 16 which are separate (unattached) and independent over a substantial length of the catheter assembly 10 (over the entire longitudinal length generally denoted by Arrow "B" in Figure 8). Figure 8b illustrates a cross section of the first and the second distal end tubes 14, 16, showing circular outer walls 44 for the first and the second distal end tubes 14, 16, as well as circular outer walls 39 for the first and the second lumens 24, 26.

The catheter assembly 10 of Figure 8 also includes a proximal portion of the first and the second distal end tubes 14, 16 with an outer configuration having a smooth, rounded, and generally oval shaped perimeter. As shown in Figure 8a, the cross section of the catheter assembly 10 over the Arrow "A" portion of the longitudinal length is generally oval in shape, Figure 8a illustrating the generally oval shaped outer wall 38 and the circular shaped outer walls 39 of the first and the second lumens 24, 26. Further, the cross section of the Arrow "A" portion of the catheter assembly 10 could include a single, oval shaped portion (as shown in Figure 8a) or two semi-circular ("D" shaped) distal end tubes 14, 16, placed back to back (i.e., with the flat portions of the semi-circle adjoining, as shown in Figure 2e and 4b) with the flat portions permanently bonded to one another. The hub 18 of the catheter assembly 10 of Figure 8 is the hub 18 detailed in Figure 3.

### A Seventh Embodiment

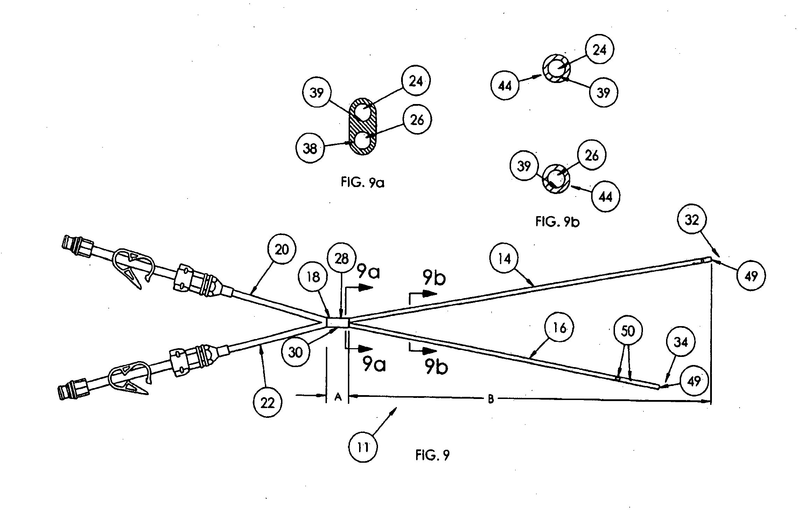

Figure 9 illustrates a catheter assembly 11, which is not part of the present invention. The catheter assembly 11 of Figure 9 is similar to the catheter assembly 10 of Figure 8, distinguished only by incorporation of an alternative hub 18. It is envisioned that the present invention could incorporate any hub known in the art, any hub described herein, or any hub to be developed.

### General & Alternative Aspects of the present invention

The present invention provides aspects of a multiple catheter assembly, having two freely and independently movable distal ends 32, 34, while also providing aspects of a single insertion method and an ability to easily manipulate a proximal portion of the unitary catheter 12 with only one tunneling procedure. The unitary catheter 12, having a smooth, generally convex exterior surface passes easily through an insertion site. A vessel wall can easily seal around the smooth, curved surface of the unitary catheter 12. If round, individual distal end tubes 14, 16 are located at the insertion site, the vessel wall will not seal as easily, or will not be capable of sealing as tightly, as if a single, rounded, generally oval or circular unitary catheter 12 type configuration is used, as a potential risk of leakage exists around and between multiple, rounded distal end tubes due to their "figure-8" configuration.

The releasably attached (i.e., splittable) portion of the catheter assemblies (e.g., the Arrow "B1" portion of Figure 6, the portion between the transition point 36 and the bonding point 48) provides, for any given patient, further adaptability and flexibility of use and insertion of the respective catheter assembly. The height and weight of the patient each has consequences effecting what a desirable length of a unitary catheter type portion of the catheter assembly would be, in relation to the rest of the catheter assembly, to ensure that a smooth, generally oval cross sectional configuration of the catheter assembly is located at a vessel wall insertion site. The splittable portion of the catheter assembly provides flexibility, at time of catheter assembly insertion, in determining an ideal length of a smooth, generally oval cross section (such as that provided by the unitary catheter configuration of the present invention), and an ideal length of independent (i.e., separate), free floating distal end tubes.

Where releasable attachment using adhesive is employed in the present invention, the outer surfaces of the tubes are releasably joined using an adhesive having an adhesive strength, relative to the material forming the tubes, greater than the cohesive strength of the adhesive. Since the adhesive is applied as a very thin layer or coating, it is not shown in Figures. However, one of ordinary skill in the art would understand, based on this disclosure, that the adhesive is applied as a partial or complete coating on one or both of the outer walls 44 of the tubes 14, 16 such that when the tubes 14, 16 are pressed together, the outer walls 44 will adhere. As a result of using an adhesive which will adhere more strongly to the tubes than to itself, the adhesive will initially hold the desired portion of the catheter assembly together, allowing manipulation of the catheter assembly in the same manner as a unitary, multi-lumen catheter. However, upon application of opposing transverse forces to distal end portions and/or the distal ends 32, 34, the adhesive will lose cohesive strength and separate longitudinally along the catheter assembly so that the tubes 14, 16 may be at least partially longitudinally split.

The length of the split may be varied depending upon the desired application. For example, for hemodialysis-type applications in which the catheter assembly is inserted into an area to be catherized, it may be desired to split the tubes 14, 16 only to the extent necessary to facilitate independent movement within the catheterized area. In blood flow and other fluid flow applications, such partial splitting may be desired to avoid the possibility that fluid could escape the catheterized area by passing between the tubes 14, 16 of the catheter assembly. While an adhesive is described herein for providing releasable attachment, it will be understood that other suitable techniques for releasable joining of the tubes 14, 16, such as a breakable ultrasonic weld, or a thin polymeric breakable layer molded between the tubes 14, 16, or other similar structures, could be used without departing from the scope of the invention, provided the tubes 14, 16 remain splittable longitudinally with only a small amount of manual force. The use of releasable attachment, splittable layers, coatings, adhesives and/or membranes described elsewhere herein, the tubes 14, 16 will not be distorted, stretched or otherwise structurally altered during the splitting of the tubes 14, 16.

The catheter assemblies of the present invention can each have smooth, round distal ends 32, 34, which do not have protuberances on their surfaces that promote clotting. A rough external surface provides protuberances, which can be points where clotting can begin. The distal ends 32, 34, which have smooth, round exterior surfaces that float freely within the vessel, do not provide a source of clot formation. The free-floating distal ends of the present invention provide beneficial aspects over an individual catheter tube, including no tendency to suction against an inside surface of the vessel wall, which minimizes the tendency of stenosis. The catheter assemblies of the present invention also have little tendency to kink, since the unitary catheter type configuration provides a good deal of support due to a thickness of its walls and cross section, and due to the smoothness of the separate distal end tubes.

As shown in the above-referenced embodiments, the first and the second distal end tubes 14, 16, and the first and the second lumens 24, 26, have a generally circular cross section, since a circular cross section is most conducive to fluid flow properties. However other shapes, such as "D"-shaped, oval, triangular, square, elliptical, kidney-bean shaped, or other configurations, are also within the scope of the invention. Further, while the distal end tubes 14, 16 and the lumens 24, 26 are preferably identical in cross section, it is within the scope of the invention to vary the size, shape or configuration of the distal end tubes and lumens in cross section such that smaller tubes and/or lumens, or varying types of lumens and distal end tubes, may be used for other applications, such as an addition of a third, smaller lumen and corresponding distal end tube for introduction of medication.

It is further within the scope of the invention that the distal end tubes have varying diameters or distal end shapes as are known in the art or to be developed. For example, the distal end tubes may have a larger diameter proximate to the unitary catheter which transitions abruptly or gradually to a smaller diameter proximate to the distal ends of the tubes. Alternatively, a more tapered, conical or angled distal end may be provided for varying applications. However, blunt ends, preferably formed of soft durometer material are preferred for the catheter assembly and distal ends to provide comfort to the patient and to avoid vessel wall trauma and stenosis.

In addition to an end hole 49 at each distal end 32, 34, the first and the second distal end tubes 14, 16 can have a plurality of side holes 50 extending through exterior surfaces of the distal end tubes 14, 16 proximate to the distal ends 32, 34 of the first and the second lumens 24, 26 (as shown in the various figures). The side holes 50 provide additional or alternative flow paths for fluids flowing between an area outside the tubes 14, 16 to an area inside the tubes 14, 16, and vice versa. The side holes 50 can be arranged circumferentially and helically around the distal end tubes 14, 16 to provide optimal flow properties, and to avoid suctioning of the distal tubes 14, 16 against an area to be catheterized, such as a vessel wall. The side holes 50 can be of various shape, but are typically circular or oval, or of some combination thereof.

The side holes 50 may also vary in number between the shorter and longer of the distal end tubes 14, 16. In one aspect of the present invention, the catheter assembly 6 of Figure 4 includes six side holes 50 in the second distal end tube 16 which spiral on the side facing the first distal end tube 14, and five side holes 50 on the first distal end tube 14, where all side holes 50 are circular except the most proximal hole on the first distal end tube 14, which is oval. In this aspect of the present invention, the side holes 50 are located 60° apart on a 360° spiral.

The side holes 50 minimize vibratory movement of the distal end tubes 14, 16 by equalizing the disturbances of intake and return flow through the side holes 50. Minimizing vibratory movement helps prevent stenosis. The side holes 50 also provide alternative openings in the distal end tubes 14, 16 so that if flow becomes blocked at one or both of the end holes 49 at the distal ends 32, 34, dialysis can continue until a replacement catheter assembly is provided. It is to be understood that the present invention also envisions embodiments having no side holes 50, employing only end holes 49 at the distal ends 32, 34.

### Materials forming the present invention

The catheter assemblies of the present invention can be made of biocompatible plastics or elastomers, and preferably made of biocompatible elastomers. Suitable biocompatible plastics may be selected from materials such as polyurethane, polyethylene, homopolymers and copolymers of vinyl acetate such as ethylene vinyl acetate copolymer, polyvinylchlorides, homopolymers and copolymers of acrylates such as polymethylmethacrylate, polymethylmethacrylate, polymethacrylate, ethylene glycol dimethacrylate, ethylene dimethacrylate and hydroxymethyl methacrylates polyurethanes, polyvinylpyrrolidone, 2-pyrrolidone, polyacrylonitrile butadiene, polycarbonates, polyamides, fluoropolymers such as homopolymers and copolymers of polytetrafluoroethylene and polyvinyl fluoride, polystyrenes, homopolymers and copolymers of styrene acrylonitrile, cellulose acetate, homopolymers and copolymers of acrylonitrile butadiene styrene, polymethylpentene, polysulfones, polyesters, polyimides, polyisobutylene, polymethylstyrene and other similar compounds known to those skilled in the art. It should be understood that these possible biocompatible polymers are included above for exemplary purposes and should not be construed as limiting.

If a biocompatible polymeric material is used to form the unitary catheter 12, it is preferred that the distal end tubes 14, 16 and the extension tubes 20, 22 be made of polymeric material, including a polyurethane polymer or a polyolefin polymeric material, having a soft durometer, as specified below.

The extension tubes 20, 22 may be made separately from the unitary catheter 12 and the distal end tubes 14, 16, and formed of a material such as polyurethane or a polyvinyl chloride polymer or elastomer. However, it is preferred that the extension tubes 20, 22 are formed of the same material as the unitary catheter 12 and the distal end tubes 14, 16.

It is most preferred to use a biocompatible elastomer for all components of the present invention. Suitable, preferred, biocompatible elastomers for use in forming the unitary catheter 12, the distal end tubes 14, 16, and preferably the extension tubes 20, 22 include biocompatible elastomers such as medical grade silicone rubbers, polyvinyl chloride elastomers, polyolefin homopolymeric and copolymeric elastomers, urethane-based elastomers, and natural rubber or other synthetic rubbers. Preferably, the unitary catheter 12, the distal end tubes 14, 16, and the extension tubes 20, 22 are made of the elastomeric material such that they are flexible, durable, soft, and with respect to those portions inserted in the patient or tunneled, they are easily conformable to the shape of the area to be catheterized and/or the subcutaneous area. Further, these materials help to minimize risk of harm to vessel walls.

If the catheter assemblies of the present invention are used for hemodialysis applications, the unitary catheter 12, the distal end tubes 14, 16, and the extension tubes 20, 22 are most preferably formed of a soft silicone elastomer having a hardness of from about 75-A to about 85-A on a Shore durometer scale. Suitable, preferred elastomers include silicone or polyurethane elastomers, and most preferably polyurethane elastomers, such as, for example, Pelletane® from Dow Corning, or Tecothane®, Carbothane® or Tecoflex®, available from Thermetics.

All components of the present invention may also optionally be made such that they include 20% barium sulfate in the elastomer to provide radiopacity if desired. While it is preferred to have a Shore-A durometer hardness in the above Shore-A durometer range and a somewhat soft material, if a biocompatible elastomer is used, particularly for hemodialysis, it is also possible to use an elastomer having a lower Shore-A durometer hardness outside this range, particularly one of more rigid material if a particular application so requires. It is also preferred that the hub is formed of an elastomeric material, and most preferably the same material as the remaining components of the catheter. However, the hub, while preferably somewhat flexible, may also preferably be somewhat harder and more rigid, by about 5-10 points on the Shore-A durometer scale, than the other components of the catheter assemblies. It will be understood based on this disclosure that softness or rigidity may be varied for different applications.

In one aspect of the present invention, the unitary catheter 12, the distal end tubes 14, 16, and the extension tubes 20, 22 are all formed of Carbothane® of 85-A durometer. Alternatively, a preferred combination may be formed of a Tecoflex® of durometer of about 80-A for the unitary catheter 12 and the distal end tubes 14, 16, and a Pelletane® of durometer of about 80A for the hub 18 and/or the extension tubes 20, 22. The additional components for attaching to dialysis or similar equipment, including luers, connectors and the like, are preferably formed of a polymeric and/or elastomeric material such as acetal, silicone 80-A or polyvinyl chloride. However, such connectors may be formed from any suitable material known or to be developed in the art for forming such connectors and/or adapters.

### Methods of making the present invention

The present invention further includes methods for making the multilumen catheter assemblies described above. Referring now to Figures 10a and 10b, the method includes forming a unitary catheter tube 60 having a proximal portion 62, a distal portion 64, and a distal end portion 66 terminating in a distal end tip 68. The unitary catheter tube 60, as shown in Figure 10a, may be formed using any suitable heat molding process, including injection molding, expansion/compression molding, and extrusion.

In one aspect of the present invention, the unitary catheter tube 60 is formed by extrusion through a die to form internal lumens such as those shown in Figure 10a'. In this embodiment, the lumens are substantially the same and substantially identical in size and configuration. The unitary catheter tube 60, with internal longitudinally extending lumens, may also be formed by injection molding the tube 60 around metal rods which have the shape of the internal lumens.

Referring now to Figure 10b, the unitary catheter tube 60 is then split longitudinally along the distal portion 64 of the tube 60 using a sharp edge such as a hot knife or razor blade (not shown) for a pre-determined distance, depending upon the particular size desired for the catheter. In one aspect of the present invention, the unitary catheter tube 60 is split a longitudinal length equal to at least one-half the total length of the tube 60. In another aspect of the present invention, the unitary catheter tube 60 is split a longitudinal length greater than one-half of the total length of the tube 60.

The tube 60 is preferably split as evenly as possible between the two lumens along an internal septum 70 (as shown in Figures 10b and 10b'). If more than two lumens are present in the catheter assembly, the unitary catheter tube would be split equally along each internal septum, with preferably a substantially equal amount of tubing material surrounding each of the split portions of the tube.

Splitting the unitary catheter tube 60 forms a first distal end tube 72 and a second distal end tube 74. The second distal end tube 74 can then cut to size relative to the first distal end tube 72, if it is desired that one distal end tube be greater in length than the other. Separate lengths for the distal end tubes helps avoid recirculation of fluids entering and leaving the tubes within the area to be catheterized.

After the unitary catheter tube 60 and the distal end tubes 72, 74 are formed, the exterior surface of the unitary catheter tube 60 and the exterior surfaces of the distal end tubes 72, 74 are then ground and polished to a smooth surface. Radio frequency (RF) tipping can be used to provide the smooth surface. Radio frequency (RF) tipping uses RF energy to re-heat an outer surface until there is some melting and then to polish the surface.

Further, the unitary catheter tube 60 and the distal end tubes 72, 74 could undergo radio frequency (RF) tipping on a mandrel, so that the tubes may be reshaped to have a generally circular transverse cross section both in the interior passageways (lumens) and on the exterior surfaces, if desired. In one aspect of the invention (referring to Figure 1), the exterior surfaces of the distal end tubes are rounded to a circular cross section from the transition point 36 to the distal ends 32, 34 (the Arrow "B" portion). In another aspect of the invention (referring to Figure 4), the exterior surfaces of the distal end tubes are rounded to a circular cross section from the bonding point 48 to the distal ends 32, 34 (the Arrow "B2" portion).

Once the surfaces are shaped and smoothed, holes can then be formed in the distal end tubes, if desired, using techniques well known in the art. The number, size, shape, and spacing of the holes are as individually preferred, but some general and specific aspects have been described above.

Portions of the split catheter can now be releasably attached, if desired, by bonding portions of exterior surfaces of the distal end tubes with a weak adhesive. In one aspect of the invention (referring to Figure 1), portions of the exterior surfaces of the distal end tubes can be adhered (releasably attached) over a proximal part of, or the entirety of, the Arrow "B" portion of the catheter assembly 5 (i.e., over a length beginning at the transition point 36 and extending toward the distal ends 32, 34, or over an entirety of the length beginning at the transition point 36 and extending to the distal ends 32, 34). In another aspect of the invention (referring to Figure 4), portions of the exterior surfaces of the distal end tubes are adhered (releasably attached) over an entirety of the length of the tubes beginning at the transition point 36 and extending to the bonding point 48 (the Arrow "B 1" portion).

As an alternative to splitting the unitary catheter tube 60, after forming the tube 60, individual distal end tubes, which may be previously extruded or heat molded, may be fused onto the unitary catheter tube 60. The distal end tubes are formed such that they each have a respective longitudinal passageway (lumen) extending longitudinally therethrough, and may also be formed to include a plurality of holes either prior to attachment to the distal end of the unitary catheter tube 60, or after attachment to the unitary catheter tube 60. The distal end tubes can be variously shaped in cross section, as desired, with some general and specific aspects having been described above.

Each formed distal end tube is then attached to the distal end of the unitary catheter tube 60 by a suitable heat molding process, or by another form of attachment, such as adhesive, ultrasonic welding or other methods known in the art, such that the first passageway in the first distal end tube is in fluid communication with the first lumen of the unitary catheter tube and the second passageway in the second distal end tube is in fluid communication with the second lumen in the unitary catheter tube. In one aspect of the present invention, heat fusing is used to attach the distal end tubes, and the fusing may be carried out using heat applied to the unitary catheter tube and to the distal end tubing lengths in a female cavity mold to create a smooth fused portion where the tube and end tube lengths meet.

Making the catheter assembly 6 of Figure 4 would require a slightly modified process if employing a fusion of individual distal end tubes to a unitary catheter tube. The unitary catheter tube 60 with an exterior having an oval cross section could still be split, prior to fusing round distal end tubes to the distal end of the split unitary catheter tube, as that portion of the catheter assembly 6 of Figure 4 between the transition point 36 and the bonding point 48 (the Arrow "B1" portion) has an exterior having two semi-circular (double "D") cross sections, as shown in Figure 4b. Therefore, prior to fusing individual distal end tubes to the distal end of the unitary catheter tube, the unitary catheter tube would require cutting (splitting), as described above, over that distance desired for the Arrow "B1" portion of the catheter assembly 6. Alternatively, performing two fusings of tubes of different cross sections is possible, as is fusing one tube and then grinding and polishing a portion thereof, as described above.

Generally, the extension tubes 20, 22 may be provided either by extruding or molding the extension tubes initially when forming the unitary catheter tube 60 using techniques similar to those used to form the distal end tubes as described above. However, it is preferred to attach the extension tubes to a proximal end of the unitary catheter tube using a hub.

Another alternative to the methods described above for making the present invention includes arranging a first catheter tube and a second catheter tube in a substantially longitudinally parallel arrangement, preferably such that they are juxtaposed to each other; however, a gap may be present between the catheter tubes. Further, more than two catheter tubes may be used and similarly arranged. Each of the catheter tubes is preferably a single lumen catheter; however, multilumen catheters may also be used for some applications. The first and the second catheter tubes each have a respective distal end, distal end portion, and at least one lumen in each catheter tube which extends longitudinally therethrough.

An outer layer (sheet) is formed around at least a portion of the length of the exterior surfaces of the first and second catheter tubes proximal to the distal end portions of the catheters. The outer layer is preferably extruded around the catheter tubes. However, the arrangement of catheter tubes and formation of the outer layer may also be formed by extruding the catheter tubes simultaneously through dies while co-extruding the outer layer around the catheter tubes. However, the outer layer is co-extruded only over a portion of the length of the exterior surfaces of the catheter tubes proximal to the distal end portions.

Once the outer layer is formed, sufficient heat molding capability may be applied to heat mold the first and the second catheter tubes together. The lumens/passageways within the catheter tubes are generally longitudinally parallel, and the catheter tubes are heat molded together in juxtaposed relation and are fixed within,the outer layer.

The distal end portions of the catheter tubes extend outwardly and distally from the portions of the lengths of the exterior surfaces of the first and the second catheter tubes which are within the outer layer. Since the distal end portions are not connected, they are capable of free floating, independent movement. It is also within the scope of the invention to heat mold a fill material between the first and the second catheter tubes to ensure that a smooth, oval exterior surface is formed around the catheters once the outer layer is formed.

As previously described, extension tubes may be provided to the ends of the catheter tubes if the outer layer is formed to extend to the proximal ends of the catheter tubes. Alternatively, the outer layer can be formed over only a portion of the length of the catheter tubes, leaving separate and independent proximal ends extending from the area within the outer layer to form extension tubes. In this instance, the proximal end portions serving as extension tubes, in the manner of extension tubes 20, 22 shown in Figure 1, extend proximally from a proximal end of the outer layer formed around the catheter tubes to provide the unitary catheter 12 of Figure 1.

A hub is then molded around the proximal end of the outer layer and the distal end of the proximally extending catheter tubes adjacent to the outer layer. Preferably, to maintain the unitary catheter and extension tubes in place, the hub mold either has cavities to receive the tubes, or metal rods inserted through the extension tubes and lumens within the formed unitary catheter portion, to retain the shape of the lumens and hold the tubes in place. A plurality of holes may also be provided to the distal end portions of the catheter tubes.

It will be appreciated by those skilled in the art that changes can be made to the embodiments described above without departing from the broad inventive concept thereof. It is understood, therefore, that this invention is not limited to the particular embodiments enclosed, but is intended to cover modifications within the sphere and scope of the present invention as defined by the intended claims.

## Claims

1. A multilumen catheter assembly (5), comprising:
a unitary portion (12) comprising an outer wall (38), a distal end, and a proximal end;
a plurality of distal end tubes (14, 16) connected to the distal end of the unitary portion (12), each of the distal end tubes (14, 16) comprising an outer wall (44) and being capable of independent movement with respect to each other;
a separating member (40) comprising:
a longitudinal planar wall (40) bisecting the unitary portion (12) along a longitudinal plane (46); and
a split portion connected to the longitudinal planar wall (40), the split portion comprising a first flat side portion and a second flat side portion, the first and second flat side portions disposed on opposite sides of the longitudinal plane (46), the first flat side portion forming a portion of a first (14) of the plurality of distal end tubes (14, 16) and the second flat side portion forming a portion of a second (16) of the plurality of distal end tubes (14, 16); and
a plurality of lumens (24, 26) extending longitudinally through the unitary portion (12) and through respective ones of the plurality of distal end tubes (14, 16) on opposite sides of the separating member (40).

2. The multilumen catheter assembly (5) of claim 1, wherein at least a portion of the outer wall (44) of each of the first and the second distal end tubes (14, 16) comprises an exterior having a generally semi-circular cross section.

3. The multilumen catheter assembly (5) of claim 1, wherein at least a portion the outer wall (44) of each of the first and the second distal end tubes (14, 16) comprises an exterior having a generally circular cross section.

4. The multilumen catheter assembly (5) of claim 1, wherein the outer wall (44) of each of the first and the second distal end tubes (14, 16) comprises an exterior comprising a first portion having a generally semi-circular cross section and a second portion having a generally circular cross section.

5. The multilumen catheter assembly (5) of claim 1, wherein at least a portion of the outer wall (38) of the unitary portion (12) comprises an exterior surface having a generally circular cross section.

6. The multilumen catheter assembly (5) of claim 1, wherein at least a portion of the outer wall (38) of the unitary portion (12) comprises an exterior surface having a generally oval cross section.

7. The multilumen catheter assembly (5) of claim 1, wherein at least a portion of each of the lumens (24, 26) comprises a generally semi-circular cross section.

8. The multilumen catheter assembly (5) of claim 1, wherein at least a portion of each of the lumens (24, 26) comprises a generally circular cross section.

9. The multilumen catheter assembly (5) of claim 1, wherein at least a portion of the first distal end tube (14) is releasably attached by a splittable bond to at least a portion of the second distal end tube (16).

10. The multilumen catheter assembly (5) of claim 1, wherein at least a portion of the first flat side portion of the first distal end tube (14) is releasably attached by a splittable bond to at least a portion of the second flat side portion of the second distal end tube (16).

11. The multilumen catheter assembly (5) of claim 10, wherein the splittable bond is splittable through use of minimal force.

12. The multilumen catheter assembly (5) of claim 11, wherein the minimal force is between one and five pounds of force.

13. The multilumen catheter assembly (5) of claim 1, wherein an exterior of the outer wall (44) of each of the first and the second distal end tubes (14, 16) comprises a generally "D"-shaped cross section, the "D"- shaped cross section of the first distal end tube (14) formed by the first flat side portion and a first rounded exterior portion, and the "D"-shaped cross section of the second distal end tube (16) formed by the second flat side portion and a second rounded exterior portion.

14. The multilumen catheter assembly (5) of claim 13, wherein at least a portion of the first flat side portion of the first distal end tube (14) is adjoined to at least a portion of the second flat side portion of the second distal end tube (16) by a releasable bond.

15. The multilumen catheter assembly (5) of claim 14, wherein the adjoined portions of the first and second distal end tubes (14, 16) together have an exterior that is generally round in cross section.

16. The multilumen catheter assembly (5) of claim 1, wherein the first and the second distal end tubes (14, 16) are aligned so that the tubes (14, 16) together have an exterior that is generally oval shaped in cross section.

17. The multilumen catheter assembly (5) of claim 1, wherein at least one of the first and the second distal end tubes (14, 16) is greater in length than the unitary portion (12), wherein length is measured in a longitudinal direction.

18. A method of making a multilumen catheter assembly (5), comprising the steps of :
forming a unitary catheter tube (60) comprising an outer wall (38), a distal portion (64) and a distal end portion (66) terminating in a distal end tip (68), a proximal portion (62) terminating in a proximal end, and a separating member (70) comprising a longitudinal planar wall (40) bisecting the unitary catheter tube (60) along a longitudinal plane (46) to form a first lumen (24) and a second lumen (26), each of the first and the second lumens (24, 26) extending longitudinally through the unitary catheter tube (60); and
splitting at least a portion of the longitudinal planar wall (40) of the unitary catheter tube (60) longitudinally along the longitudinal plane (46) at the distal end portion (66) of the unitary catheter tube (60) to form a first distal end tube (72) comprising a first outer wall (44) comprising a first flat side portion and a second distal end tube (74) comprising a second outer wall (44) comprising a second flat side portion, the first and second distal end tubes (72, 74) connected to an unsplit unitary portion (12) of the unitary catheter tube (60), the first and second flat side portions formed from the split portion of the longitudinal planar wall (40).

19. The method of claim 18, further comprising the step of releasably attaching at least a portion of the first flat side portion of the first distal end tube (72) to at least a portion of the second flat side portion of the second distal end tube (74) to form a releasable attachment, whereby the first and the second distal end tubes (72, 74) are splittable from one another by minimal force over the releasable attachment.

20. The method of claim 19, wherein the releasable attachment begins at a point (36) where the first and the second distal end tubes (72, 74) begin to extend from the unitary portion (12) and continues over a proximal portion of their longitudinal lengths, and wherein the first and second distal end tube (72, 74) are separate over a distal portion (64) of their longitudinal lengths to the distal end (68).

21. The method of claim 18, further comprising the step of finishing an exterior of each of the first and second outer walls (44) of the first and the second distal end tubes (72, 74) so that a cross section of the exterior of each of the first and second outer walls (44) is generally semi-circular over a portion of a longitudinal length of each of the first and the second distal end tubes (72, 74).

22. The method of claim 21, further comprising the step of releasably attaching at least a portion of the first distal end tube (72) to at least a portion of the second distal end tube (74) over the portion of the longitudinal length in which the exterior of the first and the second distal end tubes (72, 74) each has a generally semi-circular cross section.

23. The method of claim 18, wherein the step of splitting further comprises:
creating a point of transition (36) between split and unsplit portions of the unitary catheter tube (60).

24. The method of claim 23, further comprising the step of:
releasably attaching the first and the second distal end tubes (72, 74) to one another along a partial portion of their longitudinal lengths, the first and the second distal end tubes (72, 74) being releasably attached from the transition point (36) to a bonding point (48) located between the transition point (36) and the distal end tip (68), whereby the first and the second distal end tubes (72, 74) are splittable by minimal force from the transition point (36) to the bonding point (48) and independent and free floating from the bonding point (48) to the distal end tip (68).

25. The method of claim 23, wherein a length of the split portion of the unitary catheter tube (60), defined as a length from the transition point (36) to the distal end tip (68), is greater than a length of the unsplit unitary portion (12) of the unitary catheter tube (60) from the proximal end to the transition point (36).

26. The method of claim 18, wherein, after splitting, a length of at least one of the first and the second distal end tubes (72, 74) is greater than a length of the unitary portion (12).

27. The method of claim 18, further comprising the step of grinding and polishing the first and the second distal end tubes (72, 74) to provide a generally smooth exterior surface to each of the first and the second distal end tubes (72, 74).

28. The method of claim 27, wherein at least a portion of the exterior surface of each of the first and the second distal end tubes (72, 74) is circular in cross section after the grinding and polishing.

29. The method of claim 18, wherein forming the unitary catheter tube (60) comprises forming the unitary catheter tube (60) by a heat molding process.

30. The method of claim 29, wherein the heat molding process is extrusion.

31. The method of claim 18, wherein an exterior surface of the outer wall (38) of the unitary catheter tube (60) comprises a generally circular cross section.

32. The method of claim 18, wherein at least a portion of each of the first lumen (24) and the second lumen (26) comprises a semi-circular cross section.

33. The method of claim 18, wherein at least a portion of an exterior of each of the first outer wall (44) of the first distal end tube (72) and the second outer wall (44) of the second distal end tube (74) comprises a generally semi-circular cross section.

## Patentansprüche

1. Mehrlumen-Katheteranordnung (5), umfassend:
- einen einheitlichen Bereich (12) mit einer Außenwand (38) einem distalen Ende und einem proximalen Ende;
- eine Vielzahl distaler Endrohre (14, 16), die mit dem distalen Ende des einheitlichen Bereichs (12) verbunden sind, von denen jedes eine Außenwand (44) aufweist und die voneinander unabhängig beweglich sind;
- ein Trennelement (40) mit
einer flachen Längswand (40), die den einheitlichen Bereich (12) längs einer Längsebene (46) halbiert und
einem Spaltungsbereich, der mit der flachen Längswand (40) verbunden ist, mit einem ersten flachen Seitenteil und einem zweiten flachen Seitenteil, welche Seitenteile auf entgegengesetzten Seiten der Längsebene (46) angeordnet sind, wobei der erste flache Seitenteil einen Teil eines ersten (14) der Vielzahl distaler Endrohre (14, 16) bildet und der zweite flache Seitenteil einen Teil eines zweiten (14) der Vielzahl distaler Endrohre (14, 16) bildet;
sowie
- eine Vielzahl von Lumen (24, 26), die sich längs durch den einheitlichen Bereich (12) und jeweils durch eines der Vielzahl distaler Endrohre (14, 16) auf entgegengesetzten Seiten des Trennelements (40) erstrecken.

2. Mehrlumen-Katheteranordnung (5) nach Anspruch 1, bei welcher zumindest ein Teil der Außenwand (44) sowohl des ersten als auch des zweiten distalen Endrohrs (14, 16) eine Äußeres mit im Wesentlichen halbkreisförmigem Querschnitt aufweist.

3. Mehrlumen-Katheteranordnung (5) nach Anspruch 1, bei welcher zumindest ein Teil der Außenwand (44) sowohl des ersten als auch des zweiten distalen Endrohrs (14, 16) eine Äußeres mit im Wesentlichen kreisförmigem Querschnitt aufweist.

4. Mehrlumen-Katheteranordnung (5) nach Anspruch 1, bei welcher die Außenwand (44) sowohl des ersten als auch des zweiten distalen Endrohrs (14, 16) ein Äußeres mit einem ersten Teilbereich mit im Wesentlichen halbkreisförmigem Querschnitt und einen zweiten Teilbereich mit im Wesentlichen kreisförmigem Querschnitt aufweist.

5. Mehrlumen-Katheteranordnung (5) nach Anspruch 1, bei welcher zumindest ein Teil der Außenwand (38) des einheitlichen Bereichs (12) eine äußere Oberfläche mit im Wesentlichen kreisförmigem Querschnitt aufweist.

6. Mehrlumen-Katheteranordnung (5) nach Anspruch 1, bei welcher zumindest ein Teil der Außenwand (38) des einheitlichen Bereichs (12) eine äußere Oberfläche mit im Wesentlichen ovalem Querschnitt aufweist.

7. Mehrlumen-Katheteranordnung (5) nach Anspruch 1, bei welcher zumindest ein Teilbereich von jedem der Lumen (24, 26) im Wesentlichen halbkreisförmigen Querschnitt aufweist.

8. Mehrlumen-Katheteranordnung (5) nach Anspruch 1, bei welcher zumindest ein Teilbereich von jedem der Lumen (24, 26) im Wesentlichen kreisförmigen Querschnitt aufweist.

9. Mehrlumen-Katheteranordnung (5) nach Anspruch 1, bei welcher zumindest ein Teilbereich des ersten distalen Endrohrs (14) an zumindest einem Teilbereich des zweiten distalen Endrohrs (16) durch eine spaltbare Verbindung lösbar angebracht ist.

10. Mehrlumen-Katheteranordnung (5) nach Anspruch 1, bei welcher zumindest ein Teil des ersten flachen Seitenteils des ersten distalen Endrohrs (14) an zumindest einem Teil des zweiten flachen Seitenteils des zweiten distalen Endrohrs (16) durch eine spaltbare Verbindung lösbar angebracht ist.

11. Mehrlumen-Katheteranordnung (5) nach Anspruch 10, bei welcher die spaltbare Verbindung durch geringste Kraftaufwendung spaltbar ist.

12. Mehrlumen-Katheteranordnung (5) nach Anspruch 11, bei welcher die geringste Kraftaufwendung zwischen einem und fünf Pound-Force liegt.

13. Mehrlumen-Katheteranordnung (5) nach Anspruch 1, bei welcher ein Äußeres der Außenwand (44) sowohl des ersten als auch des zweiten distalen Endrohrs (14, 16) einen im Wesentlichen D-förmigem Querschnitt aufweist, wobei der D-förmige Querschnitt des ersten distalen Endrohrs (14) von dem ersten flachen Seitenteil und einem ersten gerundeten Außenteil gebildet wird und der D-förmige Querschnitt des zweiten distalen Endrohrs (16) von dem zweiten flachen Seitenteil und einem zweiten gerundeten Außenteil gebildet wird.

14. Mehrlumen-Katheteranordnung (5) nach Anspruch 13, bei welcher zumindest ein Teil des ersten flachen Seitenteils des ersten distalen Endrohrs (14) an zumindest einem Teil des zweiten flachen Seitenteils des zweiten distalen Endrohrs (16) über eine lösbare Verbindung anliegt.

15. Mehrlumen-Katheteranordnung (5) nach Anspruch 14, bei welcher die aneinander grenzenden Bereiche des ersten und des zweiten distalen Endrohrs (14, 16) zusammen ein Äußeres haben, dessen Querschnitt im Wesentlichen rund ist.

16. Mehrlumen-Katheteranordnung (5) nach Anspruch 1, bei welcher bei welcher das erste und das zweite distale Endrohr (14, 16) zueinander so ausgerichtet sind, dass die Rohre (14, 16) zusammen ein Äußeres haben, das im Wesentlichen ovale Querschnittsform hat.

17. Mehrlumen-Katheteranordnung (5) nach Anspruch 1, bei welcher zumindest eines des ersten und des zweiten distalen Endrohrs (14, 16) eine größere Länge hat als der einheitliche Bereich (12), wobei die Länge entlang einer Längsrichtung gemessen wird.

18. Verfahren zum Erzeugen einer Mehrlumen-Katheteranordnung (5) mit folgenden Schritten:
Ausbilden eines einheitlichen Katheterrohres (60) mit einer Außenwand (38), einem distalen Bereich (64) und einem in einer distalen Endspitze (68) auslaufenden distalen Endbereich (66), einem in einem proximalen Ende endenden proximalen Bereich (62) und einem Trennelement (40) mit einer flachen Längswand (40), die das einheitlichen Katheterrohr (60) längs einer Längsebene (46) halbiert, wodurch ein erstes Lumen (24) und ein zweites Lumen (26) gebildet wird, welche beide sich längs durch das einheitliche Katheterrohr (60) erstrecken; und
Aufspalten zumindest eines Teilbereichs der flachen Längswand (40) des einheitlichen Katheterrohres (60) längs entlang der Längsebene (46) an dem distalen Endbereich (66) des einheitlichen Katheterrohres (60), um ein erstes distales Endrohr (72) mit einer ersten Außenwand (44) mit einem ersten flachen Seitenteil und ein zweites distales Endrohr (74) mit einer zweiten Außenwand (44) mit einem zweiten flachen Seitenteil zu bilden, wobei das erste und das zweite distale Endrohr (72, 74) mit einem ungespaltenen einheitlichen Bereich (12) des einheitlichen Katheterrohres (60) verbunden sind, wobei der erste und der zweite flache Seitenteil aus den Spaltungsbereichen der flachen Längswand (40) gebildet werden.

19. Verfahren nach Anspruch 18, ferner umfassend den Schritt des lösbaren Befestigens zumindest eines Teils des ersten flachen Seitenteils des ersten distalen Endrohrs (72) an zumindest einem Teil des zweiten flachen Seitenteils der zweiten distalen Endrohrs (74) zum Ausbilden einer lösbaren Befestigung, wodurch das erste und das zweite distale Endrohr (72, 74) voneinander durch geringste Kraft über die lösbare Befestigung spaltbar sind.

20. Verfahren nach Anspruch 19, bei welchem die lösbare Befestigung an einem Punkt (36) beginnt, wo das erste und das zweite distale Endrohr (72, 74) anfangen, sich von dem einheitlichen Bereich (12) zu erstrecken, und sich über einen proximalen Bereich deren längsverlaufender Länge fortsetzt, und bei welchem das erste und das zweite distale Endrohr (72, 74) über einen distalen Bereich (64) ihrer längsverlaufenden Längen bis zum distalen Ende (68) getrennt sind.

21. Verfahren nach Anspruch 18, ferner umfassend den Schritt der Behandlung eines Äußeren sowohl der ersten als auch der zweiten Außenwand (44) des ersten und des zweiten distalen Endrohrs (72, 74), sodass ein Querschnitt des Äußeren sowohl der ersten als auch der zweiten Außenwand (44) über einen Teil der längsverlaufenden Länge sowohl des ersten als auch des zweiten distalen Endrohrs (72, 74) im Wesentlichen halbkreisförmig ist.

22. Verfahren nach Anspruch 21, ferner umfassend den Schritt des lösbaren Befestigens zumindest eines Teilbereichs des ersten distalen Endrohrs (72) an zumindest einem Teilbereich des zweiten distalen Endrohrs (74) über den Teil der längsverlaufenden Länge, in dem das Äußere des ersten und des zweiten Endrohres (72, 74) jeweils im Wesentlichen halbkreisförmigen Querschnitt hat.

23. Verfahren nach Anspruch 18, bei welchem der Schritt des Aufspaltens ferner umfasst: Schaffen eines Übergangspunktes (36) zwischen gespaltenen und ungespaltenen Bereichen des einheitlichen Katheterrohres (60).

24. Verfahren nach Anspruch 23, ferner umfassend folgenden Schritt:
lösbares Befestigen des ersten und des zweiten distalen Endrohrs (72, 74) aneinander entlang eines Teilbereichs ihrer längsverlaufenden Längen, wobei das erste und das zweite distale Endrohr (72, 74) von dem Übergangspunkt (36) bis zu einem Verbindungspunkt (48), der zwischen dem Übergangspunkt (36) und der distalen Endspitze (68) liegt, lösbar befestigt werden, wodurch das erste und das zweite distale Endrohr (72, 74) durch geringste Kraft von dem Übergangspunkt (36) bis zu dem Verbindungspunkt (48) spaltbar und von dem Verbindungspunkt (48) bis zu der distalen Endspitze (68) unabhängig und frei schwebend sind.

25. Verfahren nach Anspruch 23, bei welchem die Länge der gespaltenen Teils des einheitlichen Katheterrohres (60), definiert als die Länge von dem Übergangspunkt (36) bis zu der distalen Endspitze (68), größer ist als die Länge des ungespaltenen einheitlichen Bereichs (12) des einheitlichen Katheterrohres (60) von dem proximalen Ende bis zum Übergangspunkt (36).

26. Verfahren nach Anspruch 18, bei welchem nach dem Aufspalten eine Länge von zumindest einem des ersten und des zweiten distalen Endrohrs (72, 74) größer ist als die Länge des einheitlichen Bereichs (12).

27. Verfahren nach Anspruch 18, ferner umfassend den Schritt des Schleifens und Polierens des ersten und des zweiten distalen Endrohrs (72, 74), um diese beiden distalen Endrohre (72, 74) mit einer im Wesentlichen glatten äußeren Oberfläche zu versehen.

28. Verfahren nach Anspruch 17, bei welchem zumindest ein Teil der äußeren Oberfläche sowohl des ersten als auch des zweiten distalen Endrohrs (72, 74) nach dem Schleifen und Polieren kreisförmigen Querschnitt hat.

29. Verfahren nach Anspruch 18, bei welchem das Ausbilden des einheitlichen Katheterrohres (60) Formen des einheitlichen Katheterrohres (60) mittels eines Gussprozesses unter Wärmeanwendung umfasst.

30. Verfahren nach Anspruch 29, bei welchem der Gussprozess unter Wärmeanwendung Extrusion ist.

31. Verfahren nach Anspruch 18, bei welchem eine äußere Oberfläche der Außenwand (38) des einheitlichen Katheterrohres (60) einen im Wesentlichen kreisförmigen Querschnitt aufweist.

32. Verfahren nach Anspruch 18, bei welchem zumindest ein Teilbereich sowohl des ersten Lumens (24) als auch des zweiten Lumens (26) einen im Wesentlichen halbkreisförmigen Querschnitt aufweist.

33. Verfahren nach Anspruch 18, bei welchem zumindest ein Teil eines Äußeren sowohl der ersten Außenwand (44) des ersten distalen Endrohrs (72) als auch der zweiten Außenwand (44) des zweiten distalen Endrohrs (74) einen im Wesentlichen halbkreisförmigen Querschnitt aufweist.

## Revendications

1. Ensemble cathéter multi-lumière (5), comprenant :
une portion unitaire (12) comprenant une paroi extérieure (38), une extrémité distale, et une extrémité proximale ;
une pluralité de tubes d'extrémité distale (14, 16) reliés à l'extrémité distale de la portion unitaire (12), chacun des tubes d'extrémité distale (14, 16) comprenant une paroi extérieure (44) et étant capables d'un mouvement indépendant l'un par rapport à l'autre ;
un élément de séparation (40) comprenant :
une paroi planaire longitudinale (40) coupant en deux la portion unitaire (12) suivant un plan longitudinal (46) ; et
une portion dissociée reliée à la paroi planaire longitudinale (40), la portion dissociée comprenant une première portion latérale plane et une seconde portion latérale plane, les première et seconde portions latérales planes étant disposées sur des côtés opposés du plan longitudinal (46), la première portion latérale plane formant une portion d'un premier (14) de la pluralité de tubes d'extrémité distale (14, 16) et la seconde portion latérale plane formant une portion d'un second (16) de la pluralité de tubes d'extrémité distale (14, 16) ; et une pluralité de lumières (24, 26) s'étendant longitudinalement à travers la portion unitaire (12) et à travers certains tubes respectifs de la pluralité de tubes d'extrémité distale (14, 16) sur des côtés opposés de l'élément de séparation (40).

2. Ensemble cathéter multi-lumière (5) selon la revendication 1, dans lequel au moins une portion de la paroi extérieure (44) de chacun des premier et second tubes d'extrémité distale (14, 16) comprend un extérieur ayant une section transversale généralement semi-circulaire.

3. Ensemble cathéter multi-lumière (5) selon la revendication 1, dans lequel au moins une portion de la paroi extérieure (44) de chacun des premier et second tubes d'extrémité distale (14, 16) comprend un extérieur ayant une section transversale généralement circulaire.

4. Ensemble cathéter multi-lumière (5) selon la revendication 1, dans lequel la paroi extérieure (44) de chacun des premier et second tubes d'extrémité distale (14, 16) comprend un extérieur comprenant une première portion ayant une section transversale généralement semi-circulaire et une seconde portion ayant une section transversale généralement circulaire.

5. Ensemble cathéter multi-lumière (5) selon la revendication 1, dans lequel au moins une portion de la paroi extérieure (38) de la portion unitaire (12) comprend une surface extérieure ayant une section transversale généralement circulaire.

6. Ensemble cathéter multi-lumière (5) selon la revendication 1, dans lequel au moins une portion de la paroi extérieure (38) de la portion unitaire (12) comprend une surface extérieure ayant une section transversale généralement ovale.

7. Ensemble cathéter multi-lumière (5) selon la revendication 1, dans lequel au moins une portion de chacune des lumières (24, 26) comprend une section transversale généralement semi-circulaire.

8. Ensemble cathéter multi-lumière (5) selon la revendication 1, dans lequel au moins une portion de chacune des lumières (24, 26) comprend une section transversale généralement circulaire.

9. Ensemble cathéter multi-lumière (5) selon la revendication 1, dans lequel au moins une portion du premier tube d'extrémité distale (14) est fixée de manière amovible par une liaison dissociable à au moins une portion du second tube d'extrémité distale (16).

10. Ensemble cathéter multi-lumière (5) selon la revendication 1, dans lequel au moins une portion de la première portion latérale plane du premier tube d'extrémité distale (14) est fixée de manière amovible par une liaison dissociable à au moins une portion de la seconde portion latérale plane du second tube d'extrémité distale (16).

11. Ensemble cathéter multi-lumière (5) selon la revendication 10, dans lequel la liaison dissociable est dissociable par l'application d'une force minimale.

12. Ensemble cathéter multi-lumière (5) selon la revendication 11, dans lequel la force minimale est comprise entre une et cinq livres de force.

13. Ensemble cathéter multi-lumière (5) selon la revendication 1, dans lequel un extérieur de la paroi extérieure (44) de chacun des premier et second tubes d'extrémité distale (14, 16) comprend une section transversale généralement en forme de « D », la section transversale en forme de « D » du premier tube d'extrémité distale (14) étant formée par la première portion latérale plane et une première portion extérieure arrondie, et la section transversale en forme de « D » du second tube d'extrémité distale (16) étant formée par la seconde portion latérale plane et une seconde portion extérieure arrondie.

14. Ensemble cathéter multi-lumière (5) selon la revendication 13, dans lequel au moins une portion de la première portion latérale plane du premier tube d'extrémité distale (14) est contiguë à au moins une portion de la seconde portion latérale plane du second tube d'extrémité distale (16) par une liaison amovible.

15. Ensemble cathéter multi-lumière (5) selon la revendication 14, dans lequel les portions contiguës des premier et second tubes d'extrémité distale (14, 16) ont ensemble un extérieur de section transversale généralement ronde.

16. Ensemble cathéter multi-lumière (5) selon la revendication 1, dans lequel les premier et second tubes d'extrémité distale (14, 16) sont alignés de sorte que les tubes (14, 16) ont ensemble un extérieur de section transversale généralement de forme ovale.

17. Ensemble cathéter multi-lumière (5) selon la revendication 1, dans lequel au moins un parmi les premier et second tubes d'extrémité distale (14, 16) présente une longueur supérieure à celle de la portion unitaire (12), la longueur étant mesurée dans une direction longitudinale.

18. Procédé de fabrication d'un ensemble cathéter multi-lumière (5), comprenant les étapes consistant à:
former un tube de cathéter unitaire (60) comprenant une paroi extérieure (38), une portion distale (64) et une portion d'extrémité distale (66) se terminant en une pointe d'extrémité distale (68), une portion proximale (62) se terminant en une extrémité proximale, et un élément de séparation (70) comprenant une paroi planaire longitudinale (40) coupant en deux le tube de cathéter unitaire (60) suivant un plan longitudinal (46) pour former une première lumière (24) et une seconde lumière (26), chacune des première et seconde lumières (24, 26) s'étendant longitudinalement à travers le tube de cathéter unitaire (60) ; et
dissocier au moins une portion de la paroi planaire longitudinale (40) du tube de cathéter unitaire (60) longitudinalement suivant le plan longitudinal (46) au niveau de la portion d'extrémité distale (66) du tube de cathéter unitaire (60) pour former un premier tube d'extrémité distale (72) comprenant une première paroi extérieure (44) comprenant une première portion latérale plane et un second tube d'extrémité distale (74) comprenant une seconde paroi extérieure (44) comprenant une seconde portion latérale plane, les premier et second tubes d'extrémité distale (72, 74) étant reliés à une portion unitaire non dissociée (12) du tube de cathéter unitaire (60), les première et seconde portions latérales planes étant formées à partir de la portion dissociée de la paroi planaire longitudinale (40).

19. Procédé selon la revendication 18, comprenant en outre l'étape consistant à fixer de manière amovible au moins une portion de la première portion latérale plane du premier tube d'extrémité distale (72) à au moins une portion de la seconde portion latérale plane du second tube d'extrémité distale (74) pour former une fixation amovible, de sorte que les premier et second tubes d'extrémité distale (72, 74) sont dissociables l'un de l'autre par l'application d'une force minimale sur la fixation amovible.

20. Procédé selon la revendication 19, dans lequel la fixation amovible commence en un point (36) où les premier et second tubes d'extrémité distale (72, 74) commencent à s'étendre depuis la portion unitaire (12) et continue sur une portion proximale de leurs longueurs longitudinales, et dans lequel les premier et second tubes d'extrémité distale (72, 74) sont séparés sur une portion distale (64) de leurs longueurs longitudinales jusqu'à l'extrémité distale (68).

21. Procédé selon la revendication 18, comprenant en outre l'étape consistant à finir un extérieur de chacune des première et seconde parois extérieures (44) des premier et second tubes d'extrémité distale (72, 74) de sorte qu'une section transversale de l'extérieur de chacune des première et seconde parois extérieures (44) est généralement semi-circulaire sur une portion d'une longueur longitudinale de chacun des premier et second tubes d'extrémité distale (72, 74).

22. Procédé selon la revendication 21, comprenant en outre l'étape consistant à fixer de manière amovible au moins une portion du premier tube d'extrémité distale (72) à au moins une portion du second tube d'extrémité distale (74) sur la portion de la longueur longitudinale sur laquelle l'extérieur des premier et second tubes d'extrémité distale (72, 74) a chacun une section transversale généralement semi-circulaire.

23. Procédé selon la revendication 18, dans lequel l'étape de dissociation comprend en outre :
la création d'un point de transition (36) entre des portions dissociée et non dissociée du tube de cathéter unitaire (60).

24. Procédé selon la revendication 23, comprenant en outre l'étape consistant à :
fixer de manière amovible les premier et second tubes d'extrémité distale (72, 74) l'un à l'autre suivant une portion partielle de leurs longueurs longitudinales, les premier et second tubes d'extrémité distale (72, 74) étant fixés de manière amovible depuis le point de transition (36) jusqu'à un point de liaison (48) situé entre le point de transition (36) et la pointe d'extrémité distale (68), de sorte que les premier et second tubes d'extrémité distale (72, 74) sont dissociables par l'application d'une force minimale depuis le point de transition (36) jusqu'au point de liaison (48) et indépendants et qu'ils flottent librement depuis le point de liaison (48) jusqu'à la pointe d'extrémité distale (68).

25. Procédé selon la revendication 23, dans lequel une longueur de la portion dissociée du tube de cathéter unitaire (60), définie en tant que longueur allant du point de transition (36) jusqu'à la pointe d'extrémité distale (68), est supérieure à une longueur de la portion unitaire non dissociée (12) du tube de cathéter unitaire (60) allant de l'extrémité proximale jusqu'au point de transition (36).

26. Procédé selon la revendication 18, dans lequel, après la dissociation, une longueur d'au moins un parmi les premier et second tubes d'extrémité distale (72, 74) est supérieure à une longueur de la portion unitaire (12).

27. Procédé selon la revendication 18, comprenant en outre l'étape consistant à meuler et polir les premier et second tubes d'extrémité distale (72, 74) pour conférer une surface extérieure généralement lisse à chacun des premier et second tubes d'extrémité distale (72, 74).

28. Procédé selon la revendication 27, dans lequel au moins une portion de la surface extérieure de chacun des premier et second tubes d'extrémité distale (72, 74) est de section transversale circulaire après le meulage et le polissage.

29. Procédé selon la revendication 18, dans lequel la formation du tube de cathéter unitaire (60) comprend la formation du tube de cathéter unitaire (60) par un procédé de moulage à chaud.

30. Procédé selon la revendication 29, dans lequel le procédé de moulage à chaud est l'extrusion.

31. Procédé selon la revendication 18, dans lequel une surface extérieure de la paroi extérieure (38) du tube de cathéter unitaire (60) comprend une section transversale généralement circulaire.

32. Procédé selon la revendication 18, dans lequel au moins une portion de chacune de la première lumière (24) et de la seconde lumière (26) comprend une section transversale semi-circulaire.

33. Procédé selon la revendication 18, dans lequel au moins une portion d'un extérieur de chacune de la première paroi extérieure (44) du premier tube d'extrémité distale (72) et de la seconde paroi extérieure (44) du second tube d'extrémité distale (74) comprend une section transversale généralement semi-circulaire.
